# EUROPEAN PATENT SPECIFICATION

(11) **EP 0 662 936 B1**
(45) Date of publication and mention of the grant of the patent: **23.10.1996**
(21) Application number: 93918729.0
(22) Date of filing: 13.08.1993
(51) Int. Cl.: C07C 1/26

(54) **PROCESS FOR CONVERTING 2-CHLOROPROPANE TO PROPYLENE**
VERFAHREN ZUR UMWANDLUNG VON 2-CHLORPROPAN IN PROPYLEN
PROCEDE DE CONVERSION DE 2-CHLOROPROPANE EN PROPYLENE

(30) Priority: 01.10.1992 US 955173
(43) Date of publication of application: 19.07.1995
(73) Proprietor: THE DOW CHEMICAL COMPANY, Midland, Michigan 48640 (US)
(72) Inventor: ITO, Larry, N., Midland, MI 48640 (US); HARLEY, A., Dale, Baton Rouge, LA 70816 (US); HOLBROOK, Michael, T., Baton Rouge, LA 70808 (US); SMITH, David, D., Baton Rouge, LA 70809 (US); MURCHISON, Craig, B., Midland, MI 48640 (US); CISNEROS, Mark, D., Baton Rouge, LA 70810 (US)
(74) Representative: Burford, Anthony Frederick
(86) International application number: US9307616
(87) International publication number: WO9407818

(56) References cited:
- DD-A- 235 630
- US-A- 2 886 605

## Description

Isopropyl chloride, or 2-chloropropane, is formed as a byproduct in allyl chloride manufacture. The conversion of this byproduct material to propylene for use in the production of allyl chloride, or for other beneficial uses or for sale is a desirable end.

Several references are presently known which relate to the conversion of 1,2-dichloropropane (or PDC) to propylene.

In DD-A-235,630 ("DE '630"), for example, PDC is converted to propylene in a catalytic gas phase reaction at temperatures ranging from 170 degrees Celsius to 450 degrees Celsius. The catalyst is described as an activated carbon which has been treated with a suspension of iron oxides and/or iron oxide hydrates, and then dried at temperatures in the range of 80 degrees to 200 degrees Celsius.

Other methods described in DE '630 include the hydrogenation of PDC to propylene at 180-250 degrees Celsius via a rhodium catalyst, and the dechlorination of PDC at normal temperatures to a mixture (9:1) of propylene and chloropropylene in the presence of a pure titanium catalyst.

The present invention provides a process for directly converting 2-chloropropane to propylene in a commercially substantial proportion (that is, at a yield (defined as the product of the conversion rate of 2-chloropropane and the selectivity to propylene, on a hydrogen chloride- and hydrogen-free basis) of at least 10 percent, but preferably at least 20 percent, and more preferably still of at least 30 percent), in which 2-chloropropane is reacted with hydrogen in the presence of a catalyst including a selected Group IB metal or metals in an elemental or compound form with a selected Group VIII metal or metals, also in an elemental or compound form.

The catalysts contemplated for use in the process of the present invention fundamentally comprise a selected Group IB metal or selected Group IB metals in elemental or compound form and a selected Group VIII metal or selected Group VIII metals in elemental or compound form on a support. Preferred catalysts will, however, consist of a combination of one or more Group IB metals in elemental or compound form with one or more Group VIII metals in elemental or compound form on a support.

Preferred catalysts will comprise platinum in elemental or compound form and copper in elemental or compound form as a Group VIII metal and a Group IB metal, respectively. More preferably, the Group IB and Group VIII metals will consist of platinum and copper in their elemental or compound forms.

In general terms, copper (as a preferred Group IB metal) can be from 0.01 to 20 percent by weight (on an elemental basis) of these preferred catalysts, with platinum (as a preferred Group VIII metal) comprising from 0.01 to 5.0 percent by weight (also on an elemental basis) of the catalyst.

More preferably, copper will be from 0.05 to 15 percent by weight of the catalyst (on an elemental basis) and platinum will be from 0.03 to 3.0 percent by weight of the catalyst. Most preferably, the copper can be from 0.1 to 10 percent by weight of the catalyst (on an elemental basis) and platinum will be from 0.05 to 1.0 percent by weight of the catalyst.

The support for the catalyst can be any of those conventionally employed in the art, but is preferably silica or carbon, with carbon being more preferred. A high surface area carbon support is especially preferred, for example, a carbon having a specific surface area in an unimpregnated condition of 200 m²/g or more, especially 400 m²/g or more, and most especially 600 m²/g or more. An example of a commercially-available carbon which has been found to be well-suited for use in the present invention is a coal-based carbon produced by Calgon Carbon Corporation under the designation "BPLF3", and may generally be characterized as having a specific surface area of 1100 m²/g to 1300 m²/g, a pore volume of 0.7 to 0.85 cm³/g, and an average pore radius of 12.3 to 14 angstroms (1.23 to 1.4 nm). Based on an X-ray fluorescence analysis of this carbon, a typical bulk composition of the BPLF3 carbon has been determined to be as follows (by weight percent): silicon, 1.5 percent; aluminum, 1.4 percent; sulfur, 0.75 percent; iron, 0.48 percent; calcium, 0.17 percent; potassium, 0.086 percent; titanium, 0.059 percent; magnesium, 0.051 percent; chlorine, 0.028 percent; phosphorus, 0.026 percent; vanadium, 0.010 percent; nickel, 0.0036 percent; copper, 0.0035 percent; chromium, 0.0028 percent; and manganese, 0.0018 percent (the remainder being carbon).

Reaction conditions will vary, for example, depending on whether the process is to be carried out in the gas phase or in the liquid phase (in either a batchwise or continuous mode). For gas phase processes, contemplated reaction pressures can range from atmospheric up to 1500 psig (10.3 MPa (gauge)), with temperatures of from 100 deg. C. to 350 deg. C., residence times of from 0.25 seconds to 180 seconds, and hydrogen to 2-chloropropane feed ratios ranging on a molar basis from 0.1:1 to 100:1. More preferably, reaction pressures will range from 5 psig (0.03 MPa (gauge)) to 500 psig (3.4 MPa (gauge)), with temperatures of from 180 deg. C. to 300 deg. C., residence times of from 0.5 seconds to 120 seconds, and hydrogen to 2-chloropropane feed ratios of from 0.5:1 to 20:1. Most preferably, reaction pressures in a gas phase process will range from 40 psig (0.28 MPa (gauge)) to 300 psig (2.1 MPa (gauge)), with temperatures of from 200 deg. C. to 260 deg. C., residence times of from 1 second to 90 seconds, and hydrogen to 2-chloropropane molar feed ratios of from 0.75:1 to 6:1. In a liquid phase process, it is anticipated that suitable reaction pressures will range from atmospheric pressure up to 3000 psig (20.6 MPa (gauge)), with temperatures of from 25 degrees Celsius to 350 degrees Celsius, residence times of from one to 30 minutes, and hydrogen to 2-chloropropane molar feed ratios of from 0.1:1 to 100:1.

Preferably the catalyst will have been pretreated by exposure to a chloride source, for example, hydrogen chloride, to improve initial selectivity to propylene over propane and to make the product stream immediately useful in an allyl chloride process (wherein impurity propane can react with chlorine to produce 1-chloropropane, which because of the similarity of its boiling point to allyl chloride's boiling point is difficult to remove therefrom), for example, or to minimize venting in a propylene oxide process of any propane in the product stream fed or recycled to the propylene oxide process. In this regard, catalysts of the present invention which have been impregnated, dried and reduced under hydrogen have been observed to produce propane with decreasing selectivity and propylene with increasing selectivity over time. The initial chloride source pretreatment is expected to substantially reduce, however, the amounts of venting or downstream processing involved in making use of the product stream in a propylene oxide process or allyl chloride process, respectively.

Catalysts that have been pretreated by exposure to a chloride source and not reduced, or which have been merely dried under nitrogen and started up (and which have been in essence treated with hydrogen chloride in situ on start up), have been observed to produce the least amount of propane initially, albeit with a substantial penalty in conversion. For this reason, it is considered that it will generally be still more preferable to both reduce and pretreat (by exposure to a chloride source) a given catalyst, as opposed to reducing the catalyst solely, or not reducing the catalyst at all in favor of chloride-source pretreatment or treatment with HCl in situ. It will also be preferred to employ hydrogen chloride in the feed to reduce coking and catalyst deactivation rates. In this regard, the rate of conversion loss is preferably no more than 0.03 percent per hour, and especially no more than 0.01 percent per hour.

### Illustrative Examples

### Examples 1-9

A bimetallic platinum/copper on carbon catalyst was prepared containing 0.9 percent by weight of copper and 0.5 percent by weight on an elemental basis of platinum, for converting 2-chloropropane to reaction products including propylene in a commercially substantial proportion. Initially H₂PtCl₆·6H₂O (J. T. Baker, Inc., Baker Analyzed Grade, 37.6 percent Pt) was dissolved in distilled, deionized water.. A portion of this stock solution was added with swirling to a 50 mL Erlenmeyer flask containing a corresponding amount of CuCl₂ (Aldrich Chemical Co., Inc., 99.999 percent purity) until the CuCl₂ was dissolved. The solution was diluted with additional distilled, deionized water, and then Calgon's BPLF3 activated carbon was added to the flask with sufficient agitation to coat the carbon fully with the solution.

The catalyst was air-dried at ambient temperatures for 18 hours and in an oven at 120 degrees Celsius for 2 hours. A catalyst charge (0.6 grams) was placed on a glass wool support in the center of a tubular reactor (1/2 inch (1.27 cm) O.D., 12 inches (30.5 cm) in length) located within an aluminum block supplied with a cartridge heater to achieve the desired reaction temperature under computer control. The catalyst was then covered with a plug of glass wool.

The charged catalyst was dried for from 8 to 24 hours at 150 degrees Celsius under a nitrogen purge, and thereafter reduced by passing hydrogen through the reactor at a flow rate of 34 mL/minute for 24 hours. The reactor temperature was then lowered to the temperature setpoint of the particular catalyst run. The reactor temperature and hydrogen gas flow were allowed to equilibrate for about 1 hour before the liquid 2-chloropropane flow was started into the apparatus.

The 2-chloropropane was pumped via a high pressure syringe pump through 1/16th inch (1.6 mm) (O.D.) Monel™ nickel alloy tubing (unless specifically noted below all of the components, tubing and fittings of the test reactor apparatus were also made of Monel™ nickel alloy (Huntington Alloys, Inco Alloys International, Inc.)) into a packed sample cylinder serving as a feed evaporator.

The 1/16th inch (1.6 mm) tubing extended almost to the center of the packed cylinder, which was heated to a vaporizing temperature of 180 degrees Celsius using electrical heat tracing. Vaporization of the 2-chloropropane feedstock was accomplished in the feed line, so that the 2-chloropropane was superheated when combined with the hydrogen feed stream. Thermocouples were used to monitor the skin temperature of the feed evaporator and the temperature of the gas exiting the feed evaporator.

The hydrogen feed stream was metered to a preheater using a Model 8249 linear mass flow controller from Matheson Gas Products, Inc. Secaucus, N.J., with the preheater consisting of a packed sample cylinder wrapped with electrical heat tracing. Thermocouples were used to monitor both the skin temperature of the preheater and the temperature of the gas exiting the preheater. The preheater temperature was set and maintained at 140 degrees Celsius.

Vaporized 2-chloropropane exiting the evaporator was mixed with the hydrogen gas from the preheater in a 2 foot (0.61 meter) long section of 1/4 inch (0.64 cm) tubing maintained at a temperature of 140 degrees Celsius. The mixed gases then were passed into and reacted within the above-mentioned tubular reactor at a pressure of 76 psig (0.52 MPa (gauge)), and at varying residence times (see Table 1 below), varying hydrogen to 2-chloropropane molar feed ratios (Table 2) or varying temperatures (Table 3).

The products from the reaction under a particular set of conditions were thereafter passed to a gas sampling valve, which provided gaseous aliquots for online gas chromatographic analysis in a Hewlett-Packard Model 5890 Series II gas chromatograph (Hewlett-Packard Company). The gas chromatograph was equipped with a flame ionization detector, and used 30 meter by 0.53 millimeter (I.D.) 100 percent methyl silicone/fused silica and 30 meter by 0.53 millimeter (I.D.) porous polymer-lined fused silica columns to separate the various reaction products. Response factors were conventionally determined by injections of gravimetrically-prepared standards of the individual reaction products. These response factors were applied in conjunction with individual peak areas and the total mols of all reaction products to determine the mol percents of individual components in the reactor effluent, and the selectivity to individual reaction products.

Selectivity to the individual reaction products was determined by dividing the number of moles of an individual reaction product by the number of moles of 2-chloropropane converted, and multiplying by 100. Percent conversion and thus individual component selectivities were calculated on a hydrogen- and hydrogen chloride-free basis.

The results from these various runs are reported in Tables 1-3, where "RT" is the residence time, "PE Sel." is the selectivity to propylene, and "PA Sel." is the selectivity to propane, and show a propylene yield in the last run of Table 3 of 43.5 percent ((49.0 x 88.81)/100):

**Table 1**

| Temp. (°C) | RT (sec) | Feed Ratio | Pct. Conv. | PE Sel. (%) | PA Sel. (%) |
|---|---|---|---|---|---|
| 235 | 3.4 | 2.8 | 18.1 | 89.94 | 9.46 |
| 235 | 4.9 | 2.8 | 22.8 | 89.84 | 9.67 |
| 235 | 6.9 | 2.8 | 29.5 | 89.53 | 10.03 |

**Table 2**

| Temp. (°C) | RT (sec) | Feed Ratio | Pct. Conv. | PE Sel. (%) | PA Sel. (%) |
|---|---|---|---|---|---|
| 235 | 4.9 | 1.4 | 15.4 | 90.16 | 9.16 |
| 235 | 4.9 | 2.8 | 22.8 | 89.84 | 9.67 |
| 235 | 4.9 | 5.6 | 31.7 | 89.57 | 10.06 |

**Table 3**

| Temp. (°C) | RT (sec) | Feed Ratio | Pct. Conv. | PE Sel. (%) | PA Sel. (%) |
|---|---|---|---|---|---|
| 235 | 4.9 | 2.8 | 22.8 | 89.84 | 9.67 |
| 250 | 4.9 | 2.8 | 38.1 | 89.34 | 10.20 |
| 260 | 4.9 | 2.8 | 49.0 | 88.81 | 10.70 |

### Examples 10-15

The procedures and apparatus described in Examples 1-9 were employed with a catalyst (prepared generally as described in Examples 1-9) which was 0.5 percent by weight of platinum, and 0.45 percent by weight of copper on the BPLF3 carbon. The results of these runs (at residence times in the range of 4.6 to 5.6 seconds, at a pressure of 76 psig (0.52 MPa (gauge)), and at various temperature and hydrogen to 2-chloropropane molar feed ratios) are as shown in Table 4.

**Table 4**

| Temp. (°C) | Feed Ratio | Pct. Conv. | PE Sel. (%) | PA Sel. (%) |
|---|---|---|---|---|
| 235 | 5.4 | 31.3 | 67.2 | 32.4 |
| 250 | 5.4 | 54.4 | 49.3 | 50.3 |
| 250 | 2.8 | 36.3 | 64.5 | 35.0 |
| 260 | 2.8 | 50.3 | 62.4 | 37.1 |
| 270 | 2.8 | 65.4 | 61.7 | 37.8 |
| 280 | 2.8 | 77.9 | 63.8 | 35.7 |

While various embodiments of the processes and catalysts of the present invention have been described and/or exemplified herein, those skilled in the art will readily appreciate that numerous changes can be made thereto which are nevertheless properly considered to be within the scope of the present invention as defined by the claims below.

## Claims

1. A process for the conversion of 2-chloropropane to propylene, characterized in that 2-chloropropane is reacted with hydrogen in the presence of a supported catalyst including one or more Group IB metals in elemental or compound form and one or more Group VIII metals in elemental or compound form.

2. A process as claimed in Claim 1, wherein hydrogen chloride is incorporated in the feed to the process.

3. A process as claimed in Claim 1 or Claim 2, wherein the catalyst consists of one or more Group IB metals in elemental or compound form and one or more Group VIII metals in elemental or compound form.

4. A process as claimed in Claim 3, wherein the one or more Group IB metals includes copper and wherein the one or more Group VIII metals includes platinum.

5. A process as claimed in Claim 4, wherein the Group IB and Group VIII metals consist of copper and platinum.

6. A process as claimed in Claim 5, wherein the catalyst comprises from 0.01 to 5.0 percent by weight of platinum on an elemental basis and from 0.01 to 20 percent by weight of copper, also on an elemental basis.

7. A process as claimed in Claim 6, wherein the catalyst comprises from 0.03 to 3.0 percent by weight of platinum on an elemental basis and from 0.05 to 5 percent by weight of copper, also on an elemental basis.

8. A process as claimed in Claim 7, wherein the catalyst comprises from 0.05 to 1.0 percent by weight of platinum on an elemental basis and from 0.1 to 10 percent by weight of copper, also on an elemental basis.

9. A process as claimed in any one of the preceding claims, wherein the catalyst support is a carbon having a specific surface area of at least 200 m²/g.

10. A process as claimed in any one of the preceding claims, wherein the catalyst support is a carbon having a specific surface area of at least 400 m²/g.

11. A process as claimed in any one of the preceding claims, wherein the catalyst support is a carbon having a specific surface area of at least 600 m²/g.

12. A process as claimed in any one of the preceding claims, wherein the catalyst has been pretreated by exposure to a chloride source.

13. A process as claimed in Claim 12, wherein the chloride source is hydrogen chloride.

14. A process as claimed in Claim 12 or Claim 13, wherein the catalyst is reduced prior to said pretreatment.

15. A process as claimed in any one of the preceding claims, wherein the reaction is conducted in the gas phase at a pressure of from atmospheric pressure up to 10.3 MPa (gauge), at a temperature of from 100 degrees Celsius to 350 degrees Celsius, a residence time of from 0.25 seconds to 180 seconds, and a hydrogen to 2-chloropropane molar feed ratio of from 0.1:1 to 100:1.

16. A process as claimed in Claim 15, wherein the reaction is conducted in the gas phase at a pressure of from 0.03 MPa (gauge) to 3.4 MPa (gauge), at a temperature of from 180 degrees Celsius to 300 degrees Celsius, a residence time of from 0.5 seconds to 120 seconds, and a hydrogen to 2-chloropropane molar feed ratio of from 0.5:1 to 20:1.

17. A process as claimed in Claim 16, wherein the reaction is conducted in the gas phase at a pressure of from 0.28 MPa (gauge) to 2.1 MPa (gauge), at a temperature of from 200 degrees Celsius to 260 degrees Celsius, a residence time of from 1 second to 90 seconds, and a hydrogen to 2-chloropropane molar feed ratio of from 0.75:1 to 6:1.

## Patentansprüche

1. Verfahren zur Umwandlung von 2-Chlorpropan in Propylen, dadurch gekennzeichnet, daß 2-Chlorpropan mit Wasserstoff in der Gegenwart eines Trägerkatalysators, umfassend eines oder mehrere Gruppe IB-Metalle in Element- oder Verbindungsform und eines oder mehrere Gruppe VIII-Metalle in Element- oder Verbindungsform, umgesetzt wird.

2. Verfahren nach Anspruch 1, worin Chlorwasserstoff im Aufgabematerial des Verfahrens enthalten ist.

3. Verfahren nach Anspruch 1 oder 2, worin der Katalysator aus einem oder mehreren Gruppe IB-Metallen in Element- oder Verbindungsform und einem oder mehreren Gruppe VIII-Metallen in Element- oder Verbindungsform besteht.

4. Verfahren nach Anspruch 3, worin das eine oder die mehreren Gruppe IB-Metalle Kupfer umfassen und worin das eine oder die mehreren Gruppe VIII-Metalle Platin umfassen.

5. Verfahren nach Anspruch 4, worin die Gruppe IB- und Gruppe VIII-Metalle aus Kupfer und Platin bestehen.

6. Verfahren nach Anspruch 5, worin der Katalysator von 0,01 bis 5,0 Gew.-Prozent Platin auf Elementbasis und von 0,01 bis 20 Gew.-Prozent Kupfer, ebenfalls auf einer Elementbasis, umfaßt.

7. Verfahren nach Anspruch 6, worin der Katalysator von 0,03 bis 3,0 Gew.-Prozent Platin auf Elementbasis und von 0,05 bis 5 Gew.-Prozent Kupfer, ebenfalls auf einer Elementbasis, umfaßt.

8. Verfahren nach Anspruch 7, worin der Katalysator von 0,05 bis 1,0 Gew.-Prozent Platin auf Elementbasis und von 0,1 bis 10 Gew.-Prozent Kupfer, ebenfalls auf einer Elementbasis, umfaßt.

9. Verfahren nach einem der vorhergehenden Ansprüche, worin das Katalysatorträgermaterial Kohlenstoff mit einer spezifischen Oberfläche von mindestens 200 m²/g ist.

10. Verfahren nach einem der vorhergehenden Ansprüche, worin das Katalysatorträgermaterial Kohlenstoff mit einer spezifischen Oberfläche von mindestens 400 m²/g ist.

11. Verfahren nach einem der vorhergehenden Ansprüche, worin das Katalysatorträgermaterial Kohlenstoff mit einer spezifischen Oberfläche von mindestens 600 m²/g ist.

12. Verfahren nach einem der vorhergehenden Ansprüche, worin der Katalysator durch Behandeln mit einem Chloridausgangsmaterial vorbehandelt wurde.

13. Verfahren nach Anspruch 12, worin das Chloridausgangsmaterial Chlorwasserstoff ist.

14. Verfahren nach Anspruch 12 oder Anspruch 13, worin der Katalysator vor der Vorbehandlung reduziert wird.

15. Verfahren nach einem der vorhergehenden Ansprüche, worin die Reaktion in der Gasphase bei einem Druck von Atmosphärendruck bis zu 10,3 MPa (Gauge), bei einer Temperatur von 100 Grad Celsius bis 350 Grad Celsius, einer Verweilzeit von 0,25 Sekunden bis 180 Sekunden und einem molaren Wasserstoff zu 2-Chlorpropan Aufgabeverhältnis von 0,1:1 bis 100:1 durchgeführt wird.

16. Verfahren nach Anspruch 15, worin die Reaktion in der Gasphase bei einem Druck von 0,03 MPa (Gauge) bis zu 3,4 MPa (Gauge), bei einer Temperatur von 180 Grad Celsius bis 300 Grad Celsius, einer Verweilzeit von 0,5 Sekunden bis 120 Sekunden und einem molaren Wasserstoff zu 2-Chlorpropan Aufgabeverhältnis von 0,5:1 bis 20:1 durchgeführt wird.

17. Verfahren nach Anspruch 16, worin die Reaktion in der Gasphase bei einem Druck von 0,28 MPa (Gauge) bis zu 2,1 MPa (Gauge), bei einer Temperatur von 200 Grad Celsius bis 260 Grad Celsius, einer Verweilzeit von 1 Sekunde bis 90 Sekunden und einem molaren Wasserstoff zu 2-Chlorpropan Aufgabeverhältnis von 0,75:1 bis 6:1 durchgeführt wird.

## Revendications

1. Procédé pour la conversion du 2-chloropropane en propylène, caractérisé en ce l'on fait réagir le 2-chloropropane avec de l'hydrogène en présence d'un catalyseur sur support comprenant un ou plusieurs métaux du groupe IB sous forme d'élément ou de composé et un ou plusieurs métaux du groupe VIII sous forme d'élément ou de composé.

2. Procédé selon la revendication 1, dans lequel on incorpore le chlorure d'hydrogène dans l'alimentation vers le procédé.

3. Procédé selon la revendication 1 ou la revendication 2, dans lequel le catalyseur se compose d'un ou plusieurs métaux du groupe IB sous forme d'élément ou de composé et d'un ou plusieurs métaux du groupe VIII sous forme d'élément ou de composé.

4. Procédé selon la revendication 3, dans lequel un ou plusieurs métaux du groupe IB comprennent le cuivre et dans lequel un ou plusieurs métaux du groupe VIII comprennent le platine.

5. Procédé selon la revendication 4, dans lequel les métaux du groupe IB et du groupe VIII sont constitués par le cuivre et le platine.

6. Procédé selon la revendication 5, dans lequel le catalyseur comprend entre 0,01 et 5,0 pourcent en poids de platine calculé en élément et entre 0,01 et 20 pourcent en poids de cuivre, également calculé en élément.

7. Procédé selon la revendication 6, dans lequel le catalyseur comprend entre 0,03 et 3,0 pourcent en poids de platine calculé en élément et entre 0,05 et 5 pourcent en poids de cuivre également calculé en élément.

8. Procédé selon la revendication 7, dans lequel le catalyseur comprend entre 0,05 et 1,0 pourcent en poids de platine calculé en élément et entre 0,1 et 10 pourcent en poids de cuivre, également calculé en élément.

9. Procédé selon l'une des revendications précédentes, dans lequel le support de catalyseur est un carbone ayant une surface spécifique d'au moins 200 m²/g.

10. Procédé selon l'une des revendications précédentes, dans lequel le support de catalyseur est un carbone ayant une surface spécifique d'au moins 400 m²/g.

11. Procédé selon l'une des revendications précédentes, dans lequel le support de catalyseur est un carbone ayant une surface spécifique d'au moins 600 m²/g.

12. Procédé selon l'une des revendications précédentes, dans lequel on a prétraité le catalyseur par exposition à une source de chlore.

13. Procédé selon la revendication 12, dans lequel la source de chlore est le chlorure d'hydrogène.

14. Procédé selon l'une des revendications 12 ou 13, dans lequel on réduit le catalyseur avant ledit prétraitement.

15. Procédé selon l'une des revendications précédentes, dans lequel la réaction est conduite en phase gazeuse à une pression comprise entre la pression atmosphérique et 10,3 MPa (au manomètre), à une température comprise entre 100 degrés Celsius et 350 degrés Celsius, avec une durée de séjour comprise entre 0,25 seconde et 180 secondes, et un rapport d'alimentation molaire hydrogène/2-chloropropane compris entre 0,1:1 et 100:1.

16. Procédé selon la revendication 15, dans lequel la réaction est conduite en phase gazeuse à une pression comprise entre 0,03 MPa (au manomètre) et 3,4 MPa (au manomètre), à une température comprise entre 180 degrés Celsius et 300 degrés Celsius, avec une durée de séjour comprise entre 0,5 seconde et 120 secondes, et un rapport d'alimentation molaire hydrogène/2-chloropropane compris entre 0,5:1 et 20:1.

17. Procédé selon la revendication 16, dans lequel la réaction est conduite en phase gazeuse à une pression comprise entre 0,28 MPa (au manomètre) et 2,1 MPa (au manomètre), à une température comprise entre 200 degrés Celsius et 260 degrés Celsius, avec une durée de séjour comprise entre 1 seconde et 90 secondes, et un rapport d'alimentation molaire hydrogène/2-chloropropane compris entre 0,75:1 et 6:1.
